# EUROPÄISCHE PATENTSCHRIFT

(11) **EP 3 522 170 B1**
(45) Veröffentlichungstag und Bekanntmachung des Hinweises auf die Patenterteilung: **31.03.2021**
(21) Anmeldenummer: 18154690.4
(22) Anmeldetag: 01.02.2018
(51) Int. Cl.: G16H 40/67, G16H 40/20

(54) **SYSTEM ZUM ALARMIEREN EINER PERSON**
SYSTEM FOR ALERTING A PERSON
SYSTÈME POUR ALERTER UNE PERSONNE

(43) Veröffentlichungstag der Anmeldung: 07.08.2019
(73) Patentinhaber: Vorwerk & Co. Interholding GmbH, 42270 Wuppertal (DE)
(72) Erfinder: Mosebach, Andrej, 44809 Bochum (DE); Holz, Christian, 44137 Dortmund (DE)
(74) Vertreter: Gille Hrabal Partnerschaftsgesellschaft mbB Patentanwälte

(56) Entgegenhaltungen:
- US-A1- 2016 035 205

## Beschreibung

Die Erfindung betrifft ein System, das eine Auswertungseinheit umfasst, die ein Eintreten eines Ereignisses ermitteln und bei Eintreten des Ereignisses eine Mehrzahl von Personen alarmieren und/oder über das eingetretene Ereignis informieren kann. Die Erfindung betrifft ferner ein Verfahren sowie ein Computerprogrammprodukt zum Ausführen des Verfahrens.

Bei der Säuglingspflege wie auch bei der Altenpflege gibt es Systeme, bei denen durch z.B. ein Babyphone oder einen mobilen Notrufknopf eine oder mehrere Aufsichtspersonen informiert und/oder alarmiert werden können. Besonders das nächtliche Alarmieren einer schlafenden Aufsichtsperson, die durch das Alarmieren geweckt wird, reduziert die nächtliche Erholung.

Die Druckschrift US2016/035205A1 beschäftigt sich mit einem System und Verfahren zur Überwachung eines Menschen, wobei ein Alarm gesendet werden kann, um nur einen von zwei Pflegepersonen zu alarmieren, wenn der Mensch aufwacht, so dass die andere Pflegeperson weiter schlafen kann.

Es ist Aufgabe der Erfindung , ein alternatives System bereitzustellen.

Die Erfindung wird durch die unabhängigen Ansprüche definiert. Vorteilhafte Ausführungsformen ergeben sich aus den Unteransprüchen.

Der Stress durch das Alarmieren kann so bei den Personen insgesamt, d.h. in Summe genommen, reduziert werden. Einerseits kann der Stress insgesamt bereits durch das bloße Treffen einer Auswahl von Personen reduziert werden, weil dadurch nicht zwangsläufig alle möglichen Personen alarmiert werden müssen.

Andererseits wird durch die Auswahl der Personen auf Basis der gemessenen Vitalparameter ein möglichst geringer Stress bei der ausgewählten und alarmierten Person verursacht, insbesondere zumindest im Vergleich zu einer weiteren Person der Mehrzahl der Personen.

Mit Vitalparameter ist eine Zustandsgröße eines Organismus einer lebendigen Person gemeint, der allgemein durch eine Maßzahl angegeben werden kann. In der Regel beschreibt ein Vitalparameter eine Grundfunktion bzw. Vitalfunktion. Ein Vitalparameter, d.h. die Maßzahl, kann mithilfe eines Sensors ermittelt werden. Herzfrequenz, Blutdruck, Körpertemperatur oder Atemfrequenz sind Beispiele für Vitalparameter.

Auf Basis eines gemessenen Vitalparameters wie z.B. des Blutdrucks kann in einer Ausgestaltung ermittelt werden, ob sich eine Person gerade in einer besonders angespannten Situation befindet. Für eine Person in einer angespannten Situation bedeutet ein Alarmieren einen erhöhten Stress im Vergleich zu einer Person, die sich gerade in keiner angespannten oder in einer weniger angespannten Situation befindet.

Auf Basis eines gemessenen Vitalparameters wie z.B. der Pulsfrequenz kann in einer Ausgestaltung ermittelt werden, ob eine Person gerade einer körperlich anstrengenden Aktivität nachgeht wie z.B. Sport treibt oder eine anstrengende häusliche Arbeit verrichtet. Eine solche Person ist normalerweise gerade beschäftigt und ein Alarmieren würde einen erhöhten Stress im Vergleich zu einer Person verursachen, die gerade keiner anstrengenden Aktivität nachgeht.

Auf Basis eines gemessenen Vitalparameters wie z.B. der Bewegungsaktivität oder der elektrischen Spannungsschwankungen an der Kopfhautoberfläche kann in einer Ausgestaltung ermittelt werden, ob sich eine Person gerade in einer besonders tiefen Schlafphase befindet. Für eine Person in einer besonders tiefen Schlafphase bedeutet ein Alarmieren einen erhöhten Stress im Vergleich zu einer Person, die sich gerade in einer Schlafphase mit geringer Schlaftiefe befindet.

Alarmieren bedeutet, dass eine Person einem Reiz ausgesetzt wird, den die Person bemerkt. Die Person soll so dazu veranlasst werden, den Grund für das Alarmieren zur Kenntnis zu nehmen und/oder eine bestimmte Aktion auszuführen. Der Grund für das Alarmieren kann ein in der Regel kurz zuvor eingetretenes Ereignis sein. Bei Eintreten des Ereignisses wird also wenigstens eine Person alarmiert. Es genügt dann das Alarmieren, damit die Person von dem Eintreten des Ereignisses Kenntnis nimmt. Ein Alarmieren erfolgt in einer Ausgestaltung so, dass selbst eine sich im Tiefschlaf befindliche Person durch das Alarmieren geweckt und Kenntnis von dem Grund für das Alarmieren nehmen kann. Der Reiz kann in einer Ausgestaltung eine gezielte Bewegung wie ein Drücken, Stoßen, Ziehen oder Vibrieren sein. Der Reiz kann in einer alternativen oder ergänzenden Ausgestaltung ein akustisches Signal oder ein durch Strom induzierter Reiz sein.

Eine Information kann in einer Ausgestaltung digital abrufbar bereitgestellt werden, z.B. auf einem Smartphone oder mithilfe einer App, also einer Anwendungssoftware, eines Smartphones, um so in anspruchsgemäßer Weise zu informieren. Eine Information kann in einer Ausgestaltung über ein Display angezeigt werden. Ein Informieren verfolgt jedoch anders als das Alarmieren nicht vordergründig das Ziel, dass die informierte Person unmittelbar zum Zeitpunkt der Bereitstellung der Information auch Kenntnis von der Information nimmt. Beispielsweise wird durch ein Informieren eine tief schlafende Person nicht geweckt. Allgemein werden bei Eintreten eines Ereignisses stets alle Personen informiert.

Ein Ereignis im Sinne der Erfindung ist zuvor festgelegt worden. Das Ereignis ist in der Auswertungseinheit hinterlegt. In einer Ausgestaltung sind mehrere unterschiedliche Ereignisse in der Auswertungseinrichtung hinterlegt.

Tritt ein Ereignis ein, so erkennt die Auswerteeinheit, dass das Ereignis eingetreten ist. Sind mehrere Ereignisse hinterlegt, so erkennt die Auswerteeinheit darüber hinaus, welches Ereignis eingetreten ist.

In einer Ausgestaltung ist das Ereignis ein eingehender Telefonanruf. Die Auswertungseinheit kann dann auf Basis der gemessenen Vitalparameter eine Person auswählen und den Anruf an beispielsweise das Mobiltelefon oder Smartphone dieser ausgewählten Person weiterleiten.

In einer Ausgestaltung ist das Ereignis ein eingehender Einsatzdienstauftrag insbesondere für sich im Bereitschaftsdienst befindliche Personen wie eine Gruppe von Ärzten im Krankenhaus oder ein Team von Feuerwehrleuten in der Feuerwehrstation. Die Auswertungseinheit kann dann auf Basis der gemessenen Vitalparameter eine Person oder eine benötigte Anzahl von Personen auswählen, die zur Annahme des Einsatzdienstauftrages alarmiert werden.

In einer Ausgestaltung ist das Ereignis eine Meldung eines Überwachungsgerätes wie z.B. einer Einbruchsüberwachung oder eines Babyphones. Die Auswertungseinheit kann dann auf Basis der gemessenen Vitalparameter eine Person, z.B. einen Elternteil, auswählen und zur Kenntnisnahme der Meldung alarmieren.

Der Ausdruck "auf Basis der gemessenen Vitalparameter" bedeutet, dass für die Auswahl der wenigstens einen Person ein Vitalparameter pro Person gemessen wird und die so gemessenen Vitalparameter durch die Auswertungseinheit ausgewertet werden. Insbesondere vergleicht die Auswertungseinheit die gemessenen Vitalparameter der einzelnen Personen miteinander. Vorliegend werden die gemessenen Vitalparameter der einzelnen Personen mit einem festgelegten Schwellwert für ein Alarmieren verglichen.

Wenn die Auswertung auf Basis der gemessenen Vitalparameter ergibt, dass alle Personen für ein Alarmieren geeignet sind, können auch alle Personen der Mehrzahl der Personen ausgewählt und alarmiert werden. Die "nur die wenigstens eine ausgewählte Person" sind dann alle Personen der Mehrzahl der Personen. Dies reduziert das Risiko, dass eine alarmierte Person keine Kenntnis von dem Alarmieren genommen hat, beispielsweise durch einen technischen Defekt.

Die "Mehrzahl der Personen" umfasst jede Person, die über einen eigenen Sensor und eine eigene Steuerungseinheit verfügt. Der Sensor kann dabei den Vitalparameter der Person messen und ein entsprechendes Sensorsignal an die Steuerungseinheit übermitteln. Die Steuerungseinheit kann wiederum ein dem Sensorsignal entsprechendes Messsignal an die Auswertungseinheit übermitteln. Im einfachsten Fall ist die Steuerungseinheit eine Datenschnittstelle. Ein Sensorsignal ist ein insbesondere analoges Signal, dessen Spannung, Stromstärke und/oder Frequenz mit dem gemessenen Vitalparameter, d.h. dessen Maßzahl, korreliert. In einer Ausgestaltung führt die Steuerungseinheit eine Signalverarbeitung des Sensorsignals durch, d.h. eine Signalumwandlung und/oder Signaländerung. Vorzugsweise erfolgen durch die Steuerungseinheit eine Analog-Digital-Umwandlung und/oder eine Signaländerung durch einen Algorithmus. Das Messsignal, das von der Steuerungseinheit an die Auswertungseinheit übermittelt wird, ist bevorzugt digital und/oder entspricht der Maßzahl des Vitalparameters.

In einer ergänzenden Ausgestaltung werden mehrere Vitalparameter gemessen, d.h. mehrere unterschiedliche Vitalparameter. Eine geeignete Person oder mehrere geeignete Personen können so besonders zuverlässig für das Alarmieren ausgewählt werden.

Eine Auswertungseinheit umfasst vorzugsweise einen Prozessor und einen Speicher mit einem Computer-Programm-Code, d.h. auf dem Speicher speicherbare Befehle. Der Prozessor, der Speicher und der Computer-Programm-Code sind so konfiguriert, dass ein Verfahren mit mehreren Verfahrensschritten durchgeführt werden kann. Durch Verfahrensschritte kann wenigstens eine Person für das Alarmieren in erfindungsgemäßer Weise ausgewählt werden.

Vorzugsweise erfolgt ein Auswählen mithilfe eines Algorithmus, der in einem Computer-Programm-Code abgebildet ist und/oder durch Verfahrensschritte ausgeführt werden kann. Insbesondere umfasst auch die Steuerungseinheit einen Prozessor und einen Speicher mit einem Computer-Programm-Code.

In einer Ausführungsform sieht das System für jede Person jeweils eine Alarmierungseinrichtung vor, die so beschaffen ist, dass nur die jeweilige Person durch die Alarmierungseinrichtung alarmiert werden kann. Bei einer Aktivierung der Alarmierungseinrichtung für ein Alarmieren einer Person wird also nur die Person gezielt alarmiert, der die Alarmierungseinrichtung zugeordnet ist. Bei einer Alarmierung der Person wird also eine andere Person nicht alarmiert. Fälle, in denen eine andere Person zufällig oder beiläufig die Alarmierung für die Person wahrnimmt, sind bei dieser Betrachtung ausgenommen. Wenn zum Beispiel das Alarmieren durch Vibration erfolgt, so spürt nur die Person die Vibration, z.B. über direkten Hautkontakt mit der Alarmierungseinrichtung. Wenn eine andere Person die Vibration lediglich akustisch wahrnimmt, wird diese andere Person somit nicht im vorliegenden Sinn alarmiert.

Durch eine Alarmierungseinrichtung, die nur die jeweilige Person alarmiert, können weitere Personen bei Eintreten des Ereignisses ungestört bleiben, selbst wenn die weitere Person oder die weiteren Personen sich in der Nähe der alarmierten Person befinden.

So können beispielsweise nicht alarmierte Ärzte, Feuerwehrleute oder Elternteile weiterschlafen, während der alarmierte Kollege oder Ehepartner durch die Alarmierung geweckt wird und entsprechende Maßnahmen in Reaktion auf das Ereignis ergreift.

Wenn die Auswertungseinheit das Eintreten eines Ereignisses ermittelt, werden nur die Alarmierungseinrichtung der ausgewählten Person oder die Alarmierungseinrichtungen der ausgewählten Personen für ein Alarmieren aktiviert.

In einer Ausführungsform sieht das System für jede Person jeweils ein Display vor, um eine Information über das eingetretene Ereignis anzuzeigen. Die alarmierte Person kann dadurch Detailinformationen über das Ereignis erhalten oder erfahren, welches von mehreren möglichen Ereignisses eingetreten ist. Die alarmierte Person kann ferner über das Display Handlungsanweisungen erhalten.

Eine jede nicht-alarmierte Person kann ebenfalls durch das ihr zugeordnete Display zu gegebener Zeit die Information über das Ereignis einsehen. Somit kann jede Person informiert werden, ohne alarmiert werden zu müssen.

In einer Ausführungsform sind Steuerungseinheit und der Sensor in einem Empfangsgerät integriert, das zum Mitführen durch die jeweilige Person vorgesehen ist. Das Empfangsgerät ist also nur einer Person der Mehrzahl der Personen eindeutig zugeordnet. Das Empfangsgerät reduziert die Anzahl der Teile und erhöht so den Bedienkomfort.

Insbesondere weist die Auswertungseinheit und das Empfangsgerät, bevorzugt die Steuerungseinheit des Empfangsgeräts, eine WLAN-Schnittstelle und/oder eine Internet-Schnittstelle auf. Das Risiko des Ausfalls einer Datenübertragung infolge von Bewegungen und z.B. einem Abreißen eines Kabels kann so vermieden werden.

In einer Ausführungsform sind in dem Empfangsgerät die Alarmierungseinrichtung und/oder das Display integriert. Das Empfangsgerät reduziert somit weiter die Anzahl der Teile.

In einer Ausführungsform weist das Empfangsgerät ein Befestigungsmittel zum Befestigen am Körper einer Person auf und/oder umfasst eine hautverträgliche Anlagefläche zum direkten Befestigen am Körper. Das Empfangsgerät kann durch das Befestigungsmittel am Körper getragen werden, so dass die Person stets zuverlässig alarmiert werden kann.

Die hautverträgliche Anlagefläche ermöglicht den Einsatz eines Sensors, der einen Vitalparameter durch direkten Hautkontakt messen kann, wie z.B. die Körpertemperatur oder elektrische Spannungsschwankungen auf der Hautoberfläche. Verschiedene Vitalparameter können so besonders zuverlässig gemessen werden.

Die hautverträgliche Anlagefläche ermöglicht den Einsatz einer Afarmierungseinrichtung, die einen Reiz unmittelbar auf die Hautoberfläche ausübt, wie z.B. eine Vibration, einen Druck oder einen elektrischen Reiz. Somit kann besonders gezielt alarmiert werden, d.h., ohne eine andere Person zu alarmieren.

In einer Ausführungsform ist das Empfangsgerät ein Armband, ein Fußband oder ein Stirnband. Das Empfangsgerät umfasst also insbesondere textile Komponenten, die dem Empfangsgerät die äußerliche Erscheinung und Funktionsweise eines Armbandes, eines Fußbandes oder eines Stirnbandes verleihen. Alternativ kann das Empfangsgerät in einem Armband, Fußband oder Stirnband integriert sein. Durch ein Armband, Fußband oder Stirnband kann das Empfangsgerät zuverlässig am Körper insbesondere unter permanenten Hautkontakt getragen werden. Ein Sensor, der einen direkten Hautkontakt für das Messen eines Vitalparameters benötigt, kann so besonders zuverlässig eingesetzt werden. Gleichsam kann somit besonders gezielt durch eine Alarmierungseinrichtung alarmiert werden, die für ein Ausüben eines Reizes unmittelbar auf die Hautoberfläche eingerichtet ist.

In einer Ausführungsform sind der Sensor und/oder die Alarmierungseinrichtung für einen unmittelbaren Hautkontakt eingerichtet. Der Sensor bzw. die Alarmierungseinrichtung stehen also im betriebsbereiten Zustand unmittelbar mit einer Hautoberfläche in Kontakt. Es kann so besonders zuverlässig und auf verschiedene Art und Weise gemessen bzw. alarmiert werden.

In einer Ausführungsform ist die Alarmierungseinrichtung ein Vibrationsgeber. Durch einen Vibrationsgeber kann eine Person gezielt alarmiert werden, ohne dass eine andere in der Nähe befindliche Person in vergleichbarer Weise alarmiert wird. Bevorzugt liegt der Vibrationsgeber mit einer hautverträglichen Anlagefläche direkt auf der Hautoberfläche der Person auf. Ein Vibrationsgeber erzeugt eine mechanische Schwingung bevorzugt mittels einer rotierenden Unwucht. Ein Vibrationsgeber erzeugt eine Vibration in einem Frequenzbereich von allgemein mindestens 20 Hz.

Einer der Sensoren ist ein Gyrometer. Ein Gyrometer dient insbesondere dem Messen einer Drehbewegung. Durch das Messen der Drehbewegung kann ein Maß für die Aktivität als ein Vitalparameter der Person ermittelt werden. Es wird eine Richtungsänderung einer Drehbewegung erfasst und/oder eine Anzahl von Richtungsänderungen pro Zeitintervall von z.B. zehn Sekunden ermittelt. Erfolgen beispielsweise mindestens sechs Richtungswechsel in einem Zehn-Sekunden-Zeitraum, so ist dies ein Hinweis auf einen Wachzustand. Wird innerhalb eines Zeitintervalls keine Richtungsänderung detektiert, befindet sich die Person in einer Tiefschlafphase oder zumindest einer relativ tiefen Schlafphase. Insbesondere wird die Drehbewegung am Handgelenk gemessen. Ein besonders zuverlässiges Ermitteln der Aktivität kann so ermöglicht werden. Alternativ oder ergänzend kann das Gyrometer zum Ermitteln einer Beschleunigung eingesetzt werden. Ergänzend zu dem Gyrometer können ein Kraftsensor, ein Kraftaufnehmer, ein Piezosensor und/oder ein Dehnungsmessstreifen eingesetzt werden.

Insbesondere kann der mindestens eine Sensor ein Feuchtigkeitssensor z.B. zum Detektieren von Schweißabsonderung z.B. zur Ermittlung einer körperlichen Anstrengung, eine Bewegungssensormatte zur Ermittlung der Aktivität, ein Pulsmesser z.B. zur Ermittlung einer körperlichen Anstrengung, ein Blutdruckmesser z.B. zur Ermittlung einer geistigen Anspannung, ein Gehirnstromsensor für EEG und/oder EKG z.B. zur Ermittlung einer Schlafphase, ein Sauerstoffmesssensor z.B. zur Ermittlung einer Schlafphase, eine Kamera z.B. zur Ermittlung der Aktivität, ein Blutzuckerspiegelsensor, ein CO₂-Messgerät für Atemluft, ein Pupillengrößen-Messgerät, ein Blinzelfrequenz-Messgerät und/oder ein Atemfrequenz-Messgerät sein.

In einer Ausführungsform vergleicht die Auswertungseinheit die gemessenen Vitalparameter der einzelnen Personen miteinander, um die wenigstens eine Person auszuwählen. Es wird dann diejenige Person ausgewählt, die im Vergleich zu der anderen Person oder den anderen Personen anhand des gemessenen Vitalparameters oder der gemessenen unterschiedlichen Vitalparameter am meisten geeignet für die Alarmierung bewertet wird. Insbesondere sind für diese Bewertung eine oder mehrere Kriterien hinterlegt.

Ein Kriterium sieht vor, diejenige Person zu alarmieren, welche die geringste Anspannung aufweist. Dabei wird der Blutdruck oder die Pulsfrequenz als Vitalparameter zugrunde gelegt. Alternativ kann die Aktivität zugrunde gelegt werden. Es wird für dieses Kriterium ein ermittelter Wachzustand vorausgesetzt. Durch das Kriterium wird die wache Person ausgewählt, die am wenigsten beschäftigt erscheint. Der Stress, der durch die Alarmierung bei einer wachen Person erzeugt wird, kann so insgesamt minimiert werden.

In einer ergänzenden Ausgestaltung sieht ein anderes Kriterium vor, diejenige Person unter schlafenden Personen auszuwählen, die sich in einer Schlafphase mit der geringsten Schlaftiefe befindet. Die Aktivität oder die elektrische Spannung an der Kopfhautoberfläche kann dabei als Vitalparameter zugrunde gelegt werden. Der Stress, der durch die Alarmierung bei einer schlafenden Person erzeugt wird, kann so insgesamt minimiert werden.

In einer Ausführungsform vergleicht die Auswertungseinheit die gemessenen Vitalparameter mit einem Schwellwert, um die wenigstens eine Person auszuwählen. Mehrere geeignete Personen können so besonders einfach ausgewählt werden. Diese Ausführungsform ist nicht Teil der vorliegenden Erfindung. Insbesondere werden alle Personen ausgewählt, deren gemessener Vitalparameter auf einen Schwellwert erreicht oder über einem Schwellwert liegt. Ist der Vitalparameter die Aktivität einer schlafenden Person, so können auf diese Weise alle Personen ausgewählt werden, die sich gerade in einer Schlafphase mit vordefiniert geringer Schlaftiefe befinden. Alternativ oder ergänzend können alle Personen ausgewählt werden, deren gemessener Vitalparameter unter einem Schwellwert liegt. Alle Personen, die auf Basis des Blutdrucks und/oder der Pulsfrequenz unter einer so vordefinierten, relativ geringen Anspannung oder Anstrengung stehen, können somit besonders einfach ermittelt und ausgewählt werden.

In einer Ausgestaltung wird eine Mindest-Anzahl auszuwählender Personen und/oder eine Höchst-Anzahl auszuwählender Personen festgelegt. Diese Ausführungsform ist nicht Teil der vorliegenden Erfindung.

Insbesondere können der Vergleich der gemessenen Vitalparameter der Mehrzahl von Personen und der Vergleich eines gemessenen Vitalparameters einer Person gemeinsam durch die Auswertungseinheit berücksichtigt werden. Eine besonders zuverlässige Auswahl der wenigstens einen Person kann so ermöglicht werden, insbesondere bei einer festgelegten Mindest-Anzahl und/oder Höchst-Anzahl auszuwählender Personen.

In einer Ausführungsform ist die Auswertungseinheit mit einem maschinellen Lernalgorithmus zur Auswahl der wenigstens einen Person ausgestattet. Anhand der Eingangsgrößen in Form der Messsignale der Steuerungseinheiten der einzelnen Personen der Mehrzahl von Personen kann die Auswertungseinheit als Ausgangsgröße die wenigstens eine Person für das Alarmieren besonders geeignet auswählen, insbesondere bei Vorsehen von mehreren unterschiedlichen Vitalparametern.

Ein maschineller Lernalgorithmus ordnet allgemein einem oder mehreren Eingangsgrößen eine Ausgangsgröße zu. Die Ausgangsgröße kann auch ein Datensatz mit mehreren ausgewählten Personen sein. Ein maschineller Lernalgorithmus ist häufig die Basis für sogenannte "künstliche Intelligenz", wobei der maschinelle Lernalgorithmus aus Erfahrung "lernt" und eigenständig Muster und Gesetzmäßigkeiten auch in unbekannten Daten "erkennt". Ein maschineller Lernalgorithmus kann durch ein sogenanntes "neuronales Netz" gebildet werden oder ein "neuronales Netz" in Form eines entsprechenden Programms umfassen. Insbesondere wird ein maschineller Lernalgorithmus durch eine Modellbildungsphase und einer anschließenden Identifikationsphase erzeugt, um schließlich in einer Anwendungsphase einen Zeitpunkt für das Eintreten einer Zustandsänderung prognostizieren zu können. Insbesondere erfolgt die Modellbildungsphase beim Hersteller. Die Identifikationsphase kann beim Hersteller und/oder beim Endnutzer erfolgen. Die Anwendungsphase erfolgt dann beim Endnutzer. Zu Testzwecken kann die Anwendungsphase beim Hersteller erfolgen.

In der Modellbildungsphase wird ein mathematisches Modell, d.h. Gleichungssystem, zur Zuordnung eines oder mehrerer Eingangsgrößen zu einer Ausgangsgröße erstellt. Ein Zusammenhang von einem oder mehreren Vitalparametern mit der Auswahl der entsprechenden Person wird dabei berücksichtigt, d.h., in dem mathematischen Gleichungssystem abgebildet. Vorzugsweise wird in der Modellbildungsphase ein dynamisches Modell und/oder Differentialgleichungssystem zur Auswahl der wenigstens einen Person auf Basis der gemessenen Vitalparameter erstellt. In dem Modell bzw. dem Differentialgleichungssystem dienen die gemessenen Vitalparameter bzw. die damit korrespondierenden Messsignale eines festgelegten Sensors oder mehrerer festgelegter Sensoren als Eingangsgröße bzw. Eingangsgrößen und die Auswahl der Person oder der Personen die Ausgangsgröße.

In der Identifikationsphase wird dem maschinellen Lernalgorithmus eine Mehrzahl von Werte-Paaren mit jeweils einer Eingangsgröße und einer Ausgangsgröße bzw. mit jeweils mehreren Eingangsgrößen und einer Ausgangsgröße zugeführt. Der maschinelle Lernalgorithmus wird auf diese Weise optimiert und der Realität angepasst. Insbesondere erfolgt eine Optimierung von Konstanten in einem Differentialgleichungssystem des maschinellen Lernalgorithmus auf Basis der zugeführten Werte-Paare. Durch das Vorsehen einer Benutzerschnittstelle zur Eingabe von Rückmeldungen durch den Benutzer kann dem maschinellen Lernalgorithmus eine Ausgangsgröße zugeführt werden. In einer Ausgestaltung ist das Eingeben einer Rückmeldung durch den Benutzer vorgesehen, beispielsweise mithilfe einer App, eines Smartphone, eines Tablet-PC und/oder einer anderen Rückmeidungseinrichtung, die mit der Auswertungseinheit verbunden ist.

In der Anwendungsphase wird der maschinelle Lernalgorithmus angewendet, um anhand der Messsignale die wenigstens eine Person auszuwählen, die dann in der oben beschriebenen Weise alarmiert wird.

Durch das Ausstatten der Auswertungseinheit mit dem maschinellen Lernalgorithmus kann ein besonders präzises Auswählen der wenigstens einen Person ermöglicht werden. Ein Verallgemeinern und Ableiten von Regelmäßigkeiten von dem Verlauf eines Vitalparameters oder mehreren Vitalparameter der einzelnen Personen für sich genommen und in im Vergleich miteinander betrachtet wird ermöglicht. Eine Anwendung, welche die Eigenheiten der jeweiligen Person und/oder der Mehrzahl der Personen insgesamt berücksichtigt, kann so erzielt werden.

Ein weiterer Aspekt der Erfindung betrifft die Verwendung des Systems nach dem eingangs beschriebenen Aspekt der Erfindung, wobei das Ereignis ein Aufwachen eines Säuglings ist und die Mehrzahl der Personen die beiden Elternteile sind. Insbesondere erfolgt die Verwendung in der Nacht und/oder während der gewöhnlichen Schlaf- und/oder Ruhephase der beiden Elternteile. Wenn der Säugling nachts wach wird, kann somit durch das System nur der Elternteil geweckt werden, der sich im Vergleich zum anderen Elternteil in einer Schlafphase mit geringerer Tiefe befindet.

Ein weiterer Aspekt der Offenbarung betrifft ein System, insbesondere nach dem eingangs beschriebenen Aspekt der Erfindung, umfassend eine Auswertungseinheit, die ein Eintreten eines Ereignisses ermitteln und bei Eintreten des Ereignisses eine Mehrzahl von Personen alarmieren und/oder über das eingetretene Ereignis informieren kann. Das System sieht für jede Person jeweils eine Steuerungseinheit und einen Sensor vor. Die Steuerungseinheit kann mit der Auswertungseinheit für einen Datenaustausch verbunden werden. Der Sensor kann einen Vitalparameter der jeweiligen Person messen. Wenn die Auswertungseinheit das Eintreten eines Ereignisses ermittelt, wird wenigstens eine Person der Mehrzahl der Personen auf Basis der gemessenen Vitalparameter ausgewählt und nur die wenigstens eine ausgewählte Person wird alarmiert.

Ein weiterer Aspekt der Offenbarung betrifft ein Verfahren, bei dem mit einem Sensor ein Vitalparameter eines Lebewesens gemessen und ein dazu korrespondierendes Messsignal ausgeben wird. Eine Auswertungseinheit wertet das Messsignal des Sensors aus und prognostiziert anhand des ausgewerteten Messsignals einen Zeitpunkt für das Eintreten einer Zustandsänderung des Lebewesens. Die Merkmale, Ausführungsformen und Wirkungen des eingangs beschriebenen Systems zur Lösung der Aufgabe beziehen sich analog auch auf dieses Verfahren.

Ein weiterer Aspekt der Erfindung betrifft ein Computerprogrammprodukt. Das Computerprogrammprodukt umfasst Befehle, die bei der Ausführung des Programms durch einen Computer diesen veranlassen, die Schritte des Verfahrens gemäß dem vorhergehenden Aspekt der Erfindung auszuführen. Insbesondere ist der Computer die Auswertungseinheit. Die Merkmale, Ausführungsformen und Wirkungen des eingangs beschriebenen Systems zur Lösung der Aufgabe beziehen sich analog auch auf dieses Computerprogrammprodukt.

Nachfolgend werden Ausführungsbeispiele der Erfindung auch anhand von Figuren näher erläutert. Merkmale der Ausführungsbeispiele und weiterer nachfolgend beschriebener alternativer oder ergänzender Ausgestaltungen können einzeln oder in einer Mehrzahl mit den beanspruchten Gegenständen kombiniert werden. Die beanspruchten Schutzbereiche sind nicht auf die Ausführungsbeispiele beschränkt.

Es zeigen:
- Figur 1:: Schematische Illustration einer exemplarischen Anwendung eines Systems zur Alarmierung einer ausgewählten Person von einer Mehrzahl Personen bei Ermitteln des Eintretens eines Ereignisses;
- Figur 2:: Schematische Übersicht über Komponenten eines Systems zur Alarmierung und deren exemplarischer Aufbau.

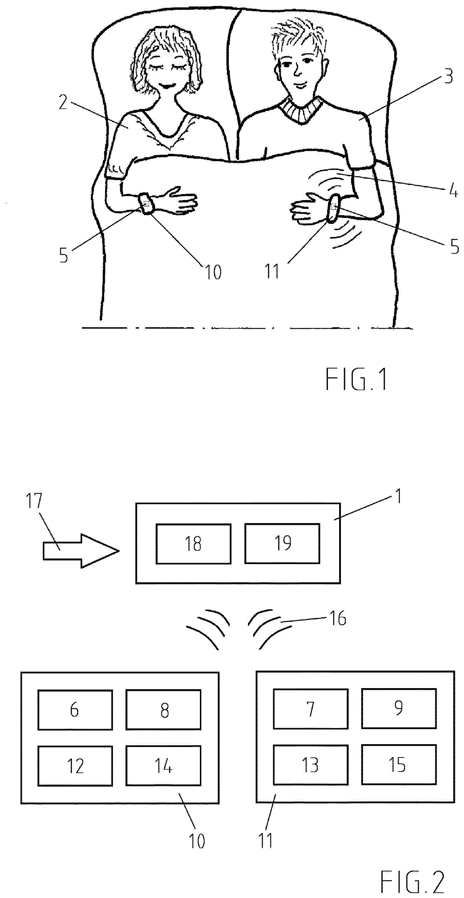

Die Figur 1 zeigt eine Person 2 und eine weitere Person 3 insbesondere während einer Ruhephase beispielsweise in der Nacht. Beide Personen 2, 3 tragen jeweils ein als Armband ausgeführtes Empfangsgerät 10, 11. Jede der Personen 2, 3 wird bei Eintreten eines Ereignisses informiert. Dazu wird eine Information 5 angezeigt. Doch nur die ausgewählte Person 3 wird bei Eintreten eines Ereignisses zusätzlich durch ein Alarmierungssignal 5 alarmiert, damit die Person 3 zeitnah Kenntnis von dem Eintreten des Ereignisses nimmt.

Die Figur 2 zeigt den Aufbau eines Systems zur Alarmierung mit einer Auswertungseinheit 1 und mehreren Empfangsgeräten 10, 11. Insbesondere handelt es sich dabei um das System der Figur 1. Jedes Empfangsgerät 10, 11 umfasst jeweils ein Display 14, 15 und/oder jeweils eine Alarmierungseinrichtung 12, 13. Die Displays 14, 15 können eine Information 5 über das eingetretene Ereignis anzeigen. Die aktivierte Alarmierungseinrichtung 13 der ausgewählten Person 3 gibt ein Alarmierungssignal 5 in Form einer Vibration für ein Alarmieren an die Person 3 ab, die dadurch aufgeweckt wird. Die Person 2 wird hingegen durch das Alarmierungssignal 5 nicht geweckt.

Jedes Empfangsgerät 10, 11 umfasst jeweils einen Sensor 8, 9 zum Messen eines Vitalparameters der jeweiligen Person 2, 3. Mit "jeweiliger Person" ist diejenige Person gemeint, die das Empfangsgerät 10, 11 trägt und somit diesem Empfangsgerät 10, 11 zugeordnet ist.

Jedes Empfangsgerät 10, 11 umfasst jeweils eine Steuerungseinheit 6, 7. Die Steuerungseinheit 6, 7 ist mit der Auswertungseinheit 1 für einen Datenaustausch verbunden. Die Verbindung ist insbesondere eine drahtlose Verbindung. Bevorzugt wird eine Funkverbindung oder WLAN-Verbindung als drahtlose Verbindung eingesetzt.

Die Auswertungseinheit 1 umfasst einen Prozessor 18 und einen Speicher 19. Auf dem Speicher 19 ist ein Computerprogramm bzw. Computerprogrammprodukt hinterlegt, durch das ein Eingangssignal 17 für das Ermitteln des Eintretens eines Ereignisses ausgewertet werden kann. In einer Ausgestaltung ist nur ein Ereignis vorgesehen, dessen Eintritt durch die Auswertungseinheit 1 ermittelt werden kann. Rechenkapazität kann so gespart werden. In einer bevorzugten Ausgestaltung sind mehrere verschiedene Ereignisse vorgesehen, deren jeweiliger Eintritt durch die Auswertungseinheit 1 ermittelt werden kann.

Der Sensor 8, 9 misst einen Vitalparameter der Person 2, 3 und übermittelt ein entsprechendes Sensorsignal an die Steuerungseinheit 6, 7.

In einer Ausgestaltung führt die Steuerungseinheit 6, 7 eine Signalverarbeitung des Sensorsignals durch, um ein Messsignal 16 für die Auswertungseinheit 1 zu erhalten. Alternativ oder ergänzend leitet die Steuerungseinheit 6, 7 das Sensorsignal an die Auswertungseinheit 1 weiter.

In einer Ausgestaltung übermittelt die Steuerungseinheit 6, 7 das verarbeitete Messsignal 16 an die Auswertungseinheit 1 insbesondere zusammen mit einer Zuordnung zu der Person 2, 3 oder des Empfangsgerätes 10, 11 der Person 2, 3, dessen Vitalparameter durch das Messsignal 16 beschrieben wird.

In einer Ausgestaltung wertet die Steuerungseinheit 6, 7 die Sensorsignale derart aus, dass das Messsignal 16 bereits angibt, ob die Person 2, 3 auszuwählen ist oder nicht. Die Auswertungseinheit 1 kann somit entlastet werden. In einer Ausgestaltung zeichnet die Steuerungseinheit 6, 7 die Sensorsignale über einen vorgegebenen Zeitraum auf, um eine Signalverarbeitung und/oder Auswertung vorzunehmen. Eine besonders zuverlässige und qualitativ hochwertige Signalverarbeitung bzw. Auswertung kann so ermöglicht werden. In einer Ausgestaltung zeichnet die Auswertungseinheit 1 die Messsignale 16 über einen vorgegebenen Zeitraum für die Auswertung auf. Eine besonders zuverlässige Auswahl der wenigstens einen Person 3 kann so ermöglicht werden.

In einer Ausgestaltung triggert die Auswertungseinheit 1 das Messen eines Vitalparameters durch den Sensor 8, 9 und/oder das Übermitteln des Messsignal 16 an die Auswertungseinheit 1. Unnötige Messvorgänge, Verarbeitungsvorgänge und Übermittlungsvorgänge können so eingespart werden.

Wenn die Auswertungseinheit 1 das Eintreten eines Ereignisses ermittelt und damit detektiert hat, wird eine Auswertung der Messsignale 16 der einzelnen Personen 2, 3 vorgenommen, um wenigstens eine Person 3 für das Alarmieren auszuwählen.

In einem ersten Beispiel ist das Eingangssignal 17 ein eingehender Anruf. Die Auswertungseinheit 1 fordert darauf hin ein aktuelles Messsignal 16 von den Empfangsgeräten 10, 11 der Mehrzahl der Personen 2, 3 an. Mittels in der Auswertungseinheit 1 hinterlegten Schwellwerte, die also in dem Speicher 19 gespeichert sind, wird wenigstens eine der Personen 2, 3 für das Alarmieren ausgewählt. Beispielsweise ist der Vitalparameter die Aktivität. Die Messsignale 16 haben gezeigt, dass die Person 2 tiefer schläft als die weitere Person 3. Gleichzeitig ist die Schlaftiefe der Person 2 unterhalb einem Schwellwert für eine Auswahl bzw. Alarmierung. Daher sendet die Auswertungseinheit 1 eine Information über den eingehenden Anruf, z.B. die Telefonnummer des Anrufers und die Zeit des Eingangs des Anrufes, an die Empfangsgeräte 10, 11 beider Personen 2, 3, damit die Information an den Displays 14, 15 angezeigt oder abgerufen werden kann. Ein Ansteuersignal zum Aktivieren der Alarmierungseinrichtung 12, 13 erhält nur das Empfangsgerät 11 der weiteren Person 3. Die Alarmierungseinrichtung 13 der weiteren Person 3 gibt insbesondere ein Vibrationsalarm ab, das bevorzugt direkt auf die Hautoberfläche der weiteren Person 3 einwirkt. Die weitere Person 3 wird so geweckt, während die Person 2 weiterschläft und nicht durch die Alarmierung der weiteren Person 3 geweckt wird. Die weitere Person 3 wird dann zum Telefon gehen und den Anruf annehmen. In einer Ausgestaltung leitet die Auswertungseinheit 1 den Anruf direkt auf das Mobiltelefon oder Smartphone oder das Empfangsgerät 11 mit Telefonfunktion weiter.

In einem zweiten Beispiel zeigt das Eingangssignal 17 an, dass ein Säugling erwacht ist oder in einem prognostizierten Zeitabstand erwachen wird. Insbesondere weist der Säugling eine Sensoreinrichtung zum Erfassen eines Vitalparameters des Säuglings auf, der mit einer Analyseeinrichtung verbunden ist. Insbesondere vermag die Analyseeinrichtung auf Basis des erfassten Vitalparameters bevorzugt durch einen maschinellen Lernalgorithmus das Erwachen des Säuglings zu ermitteln oder zu prognostizieren.

Wenn die Auswertungseinheit 1 das Eintreten des Aufwachens oder eines prognostizierten Aufwachens des Säuglings auf Basis des Eingangssignals 17 ermittelt, wird auf Basis der durch die Sensoren 8, 9 gemessenen Vitalparameter der Mehrzahl der Personen 2, 3 wenigstens eine Person 3 ausgewählt und durch das Empfangsgerät 11 alarmiert. Es braucht sich dann nur eine Person 3, also insbesondere nur ein Elternteil, um den Säugling kümmern. Die Ruhezeit für Eltern von einem Säugling kann somit insgesamt erhöht werden.

In einer Weiterentwicklung kann die Auswertungseinheit 1 ein nicht gezeigtes Gerät bzw. Empfangsgerät, insbesondere ein Haushaltsgerät, bevorzugt eine Küchenmaschine, einen Milchflaschenerhitzer oder einen Ofen bei Ermitteln des Eintretens eines Ereignisses aktivieren. Wenn das Ereignis das Aufwachen oder prognostizierte Aufwachen eines Säuglings ist, kann der alarmierte Elternteil dann eine sich bereits in der Zubereitung befindliche oder fertig zubereitete Milchflasche oder Menge Babybrei an einem dafür vorgesehenen Gerät antreffen. Der Säugling kann so schneller verpflegt werden. Gleichzeitig wird die Zeit der Unterbrechung der Ruhephase des alarmierten Elternteils reduziert.

## Patentansprüche

1. System umfassend eine Auswertungseinheit (1), die ein Eintreten eines Ereignisses ermittelt und bei Eintreten des Ereignisses eine Mehrzahl von Personen (2, 3) über das eingetretene Ereignis informieren kann, wobei das System für jede Person (2, 3) jeweils eine Steuerungseinheit (6, 7) und mindestens einen Sensor (8, 9) vorsieht, wobei die Steuerungseinheit (6, 7) mit der Auswertungseinheit (1) für einen Datenaustausch verbindbar ist und einer der mindestens ein Sensoren (8, 9) einen Vitalparameter der jeweiligen Person (2, 3) misst, wobei das System so eingerichtet ist, dass wenn die Auswertungseinheit (1) das Eintreten eines Ereignisses ermittelt, wenigstens eine Person (3) der Mehrzahl der Personen (2, 3) auf Basis der gemessenen Vitalparameter ausgewählt und nur die wenigstens eine ausgewählte Person (3) alarmiert wird, **dadurch gekennzeichnet, dass** unter den mindestens ein Sensoren (8, 9) ein Gyrometer ist, eine Anzahl von Richtungsänderungen pro Zeitintervall durch das Gyrometer zur Ermittlung eines Schlaf- oder Wachzustandes ermittelt wird und unter wachen Personen diejenige Person (2, 3) alarmiert wird, welche die geringste Anspannung aufweist, wobei als Vitalparameter der Blutdruck oder die Pulsfrequenz zugrunde gelegt wird.

2. System nach Anspruch 1, **dadurch gekennzeichnet, dass** das System für jede Person (2, 3) jeweils eine Alarmierungseinrichtung (12, 13) vorsieht, die so beschaffen ist, dass nur die jeweilige Person (3) durch die Alarmierungseinrichtung (13) alarmiert wird.

3. System nach einem der vorhergehenden Ansprüche, **dadurch gekennzeichnet, dass** das System für jede Person (2, 3) jeweils ein Display (14, 15) vorsieht, um eine Information (5) über das eingetretene Ereignis anzuzeigen, über das die Person (2, 3) informiert wurde.

4. System nach einem der vorhergehenden Ansprüche, **dadurch gekennzeichnet, dass** die Steuerungseinheit (6, 7) und der Sensor (8, 9) in einem Empfangsgerät (10, 11) integriert sind, das zum Mitführen durch die jeweilige Person (2, 3) vorgesehen ist.

5. System nach dem vorhergehenden Anspruch, **dadurch gekennzeichnet, dass** in dem Empfangsgerät (10, 11) die Alarmierungseinrichtung (12, 13) und/oder das Display (14, 15) integriert sind.

6. System nach einem der zwei vorhergehenden Ansprüche, **dadurch gekennzeichnet, dass** das Empfangsgerät (10, 11) ein Befestigungsmittel zum Befestigen am Körper einer Person (2, 3) aufweist und/oder eine hautverträgliche Anlagefläche zum direkten Befestigen am Körper umfasst.

7. System nach einem der drei vorhergehenden Ansprüche, **dadurch gekennzeichnet, dass** das Empfangsgerät (10, 11) ein Armband, ein Fußband oder ein Stirnband ist.

8. System nach einem der vorhergehenden Ansprüche, **dadurch gekennzeichnet, dass** der Sensor (8, 9) und/oder die Alarmierungseinrichtung (12, 13) für einen unmittelbaren Hautkontakt eingerichtet sind.

9. System nach einem der vorhergehenden Ansprüche, **dadurch gekennzeichnet, dass** die Alarmierungseinrichtung (12, 13) ein Vibrationsgeber ist und/oder der Sensor (8, 9) ein Gyrometer ist.

10. System nach einem der vorhergehenden Ansprüche, **dadurch gekennzeichnet, dass** die Auswertungseinheit (1) die gemessenen Vitalparameter der einzelnen Personen (2, 3) miteinander vergleicht, um die wenigstens eine Person (3) auszuwählen.

11. System nach einem der vorhergehenden Ansprüche, **dadurch gekennzeichnet, dass** die Auswertungseinheit (1) mit einem maschinellen Lernalgorithmus zur Auswahl der wenigstens einen Person (3) ausgestattet ist.

12. Verfahren, bei dem eine Auswertungseinheit (1) ein Eintreten eines Ereignisses ermittelt und bei Eintreten des Ereignisses eine Mehrzahl von Personen (2, 3) über das eingetretene Ereignis informieren kann (5), wobei für jede Person (2, 3) jeweils eine Steuerungseinheit (6, 7) und mindestens ein Sensor (8, 9) vorgesehen ist, wobei die Steuerungseinheit (6, 7) mit der Auswertungseinheit (1) für einen Datenaustausch verbindbar ist und einer der mindestens ein Sensoren (8, 9) einen Vitalparameter der jeweiligen Person (2, 3) misst, und wenn die Auswertungseinheit (1) das Eintreten eines Ereignisses ermittelt, wenigstens eine Person (3) der Mehrzahl der Personen (2, 3) auf Basis der gemessenen Vitalparameter ausgewählt und nur die wenigstens eine ausgewählte Person (3) alarmiert wird, **dadurch gekennzeichnet, dass** unter den mindestens ein Sensoren (8, 9) ein Gyrometer ist, eine Anzahl von Richtungsänderungen pro Zeitintervall durch das Gyrometer zur Ermittlung eines Schlaf- oder Wachzustandes ermittelt wird und die Auswertungseinheit (1) unter wachen Personen diejenige Person (2, 3) alarmiert, welche die geringste Anspannung aufweist, wobei als Vitalparameter der Blutdruck oder die Pulsfrequenz zugrunde gelegt wird.

13. Computerprogrammprodukt, umfassend Befehle, die bei der Ausführung des Programms durch einen Computer, insbesondere die Auswertungseinheit (1), diesen veranlassen, die Schritte des Verfahrens nach dem vorhergehenden Anspruch auszuführen.

## Claims

1. System comprising an evaluation unit (1) that determines an occurrence of an event and allows, when the event occurs, to inform a plurality of persons (2, 3) about the occurred event, wherein the system provides for each person (2, 3) a control unit (6, 7) and at least one sensor (8, 9), respectively, wherein the control unit (6, 7) can be connected to the evaluation unit (1) for a data exchange and one of the at least one sensor (8, 9) measures a vital parameter of the respective person (2, 3), wherein the system is configured such that when the evaluation unit (1) determines the occurrence of an event, at least one person (3) of the plurality of persons (2, 3) is selected based on the measured vital parameters and only the at least one selected person (3) is alerted, **characterized in that** among the at least one sensor (8, 9) is a gyrometer, a number of changes of direction per time interval is determined by the gyrometer for determining a sleeping or waking state, and among awake persons, the person (2, 3) having the lowest tension is alerted, wherein the blood pressure or the pulse rate is taken as a basis as the vital parameter.

2. System according to claim 1, **characterized in that** the system provides for each person (2, 3) a respective alert device (12, 13), which is such that only the respective person (3) is alerted by the alert device (13).

3. System according to one of the preceding claims, **characterized in that** the system provides for each person (2, 3) a respective display (14, 15) for displaying information (5) about the event that has occurred about which the person (2, 3) has been informed.

4. System according to one of the preceding claims, **characterized in that** the control unit (6, 7) and the sensor (8, 9) are integrated in a receiving device (10, 11) intended to be carried along by the respective person (2, 3).

5. System according to the preceding claim, **characterized in that** the alert device (12, 13) and/or the display (14, 15) are integrated in the receiving device (10, 11).

6. System according to one of the two preceding claims, **characterized in that** the receiving device (10, 11) comprises an attachment means for attachment to the body of a person (2, 3) and/or comprises a skin-compatible contact surface for direct attachment to the body.

7. System according to one of the three preceding claims, **characterized in that** the receiving device (10, 11) is a bracelet, a footband or a headband.

8. System according to one of the preceding claims, **characterized in that** the sensor (8, 9) and/or the alert device (12, 13) are configured for direct skin contact.

9. System according to one of the preceding claims, **characterized in that** the alert device (12, 13) is a vibration generator and/or the sensor (8, 9) is a gyrometer.

10. System according to one of the preceding claims, **characterized in that** the evaluation unit (1) compares the measured vital parameters of the individual persons (2, 3) with each other in order to select the at least one person (3).

11. System according to one of the preceding claims, **characterized in that** the evaluation unit (1) is equipped with a machine learning algorithm for selecting the at least one person (3).

12. Method in which an evaluation unit (1) determines an occurrence of an event and allows, when the event occurs, to inform (5) a plurality of persons (2, 3) about the occurred event, wherein for each person (2, 3) a control unit (6, 7) and at least one sensor (8, 9) is provided, respectively, wherein the control unit (6, 7) can be connected to the evaluation unit (1) for a data exchange and one of the at least one sensor (8, 9) measures a vital parameter of the respective person (2, 3), and when the evaluation unit (1) determines the occurrence of an event, at least one person (3) of the plurality of persons (2, 3) is selected based on the measured vital parameters and only the at least one selected person (3) is alerted, **characterized in that** among the at least one sensor (8, 9) is a gyrometer, a number of changes of direction per time interval is determined by the gyrometer for determining a sleeping or waking state, and the evaluation unit (1) alerts, among awake persons, the person (2, 3) having the lowest tension, wherein the blood pressure or the pulse rate is taken as a basis as the vital parameter.

13. Computer program product comprising instructions which, when the program is executed by a computer, in particular the evaluation unit (1), cause the latter to carry out the steps of the method according to the preceding claim.

## Revendications

1. Système comprenant une unité d'évaluation (1), qui détermine, si un évènement a lieu et si l'évènement a lieu, elle peut informer une pluralité de personnes (2, 3) de l'évènement, qui s'est produit, dans lequel le système prévoit respectivement une unité de commande (6, 7) et au moins un capteur (8, 9) pour chaque personne (2, 3), l'unité de commande (6, 7) pouvant être reliée à l'unité d'évaluation (1) pour un échange de données, et au moins un des capteurs (8, 9) mesurant un paramètre vital de la personne respective (2, 3), dans lequel le système est configuré, de sorte que si l'unité d'évaluation (1) détermine qu'un évènement s'est produit, au moins une personne (3) de la pluralité de personnes (2, 3) sera sélectionnée sur la base des paramètres vitaux mesurés et seulement la personne sélectionnée sera alertée, **caractérisé en ce qu'**il y a un gyromètre parmi les au moins un capteurs (8, 9), un nombre de changements de direction par intervalle de temps est déterminé par le gyromètre pour déterminer un état à l'éveil ou de sommeil et la personne (2, 3) parmi les personnes éveillées sera alertée, qui montre la moindre tension, dans lequel la tension artérielle ou la fréquence du pouls est prise comme base du paramètre vital.

2. Système selon la revendication 1, **caractérisé en ce que** le système prévoit respectivement un dispositif d'alerte (12, 13) pour chaque personne (2, 3), lequel dispositif d'alerte (12, 13) est configuré de sorte que seulement la personne respective (3) est alertée par le dispositif d'alerte (13).

3. Système selon l'une des revendications précédentes, **caractérisé en ce que** le système prévoit respectivement un écran (14, 15) pour chaque personne (2, 3) pour afficher une information (5) sur l'évènement, qui s'est produit, et sur lequel la personne (2, 3) a été informé.

4. Système selon l'une des revendications précédentes, **caractérisé en ce que** l'unité de commande (6, 7) et le capteur (8, 9) sont intégrés dans un appareil récepteur (10, 11), qui est prévu pour être emmené par la personne respective (2, 3).

5. Système selon la revendication précédente, **caractérisé en ce que** le dispositif d'alerte (12, 13) et/ou l'écran (14, 15) sont intégrés dans l'appareil récepteur (10, 11).

6. Système selon l'une des deux revendications précédentes, **caractérisé en ce que** l'appareil récepteur (10, 11) comprend un moyen de fixation pour le fixer au corps d'une personne (2, 3) et/ou une surface de contact douce pour la peau pour la fixation directe au corps.

7. Système selon l'une des trois revendications précédentes, **caractérisé en ce que** l'appareil récepteur (10, 11) est un bracelet, une bride de cheville ou un bandeau.

8. Système selon l'une des revendications précédentes, **caractérisé en ce que** le capteur (8, 9) et/ ou le dispositif d'alerte (12, 13) sont adaptés pour un contact dermique immédiat.

9. Système selon l'une des revendications précédentes, **caractérisé en ce que** le dispositif d'alerte (12, 13) est un vibreur et/ou le capteur (8, 9) est un gyromètre.

10. Système selon l'une des revendications précédentes, **caractérisé en ce que** l'unité d'évaluation (1) compare les paramètres vitaux mesurés des personnes individuelles (2, 3) les uns avec les autres pour sélectionner l'au moins une personne (3).

11. Système selon l'une des revendications précédentes, **caractérisé en ce que** l'unité d'évaluation (1) est équipée d'un algorithme d'apprentissage mécanique pour sélectionner l'au moins une personne (3).

12. Procédé, dans lequel une unité d'évaluation (1) détermine, si un évènement a lieu et si l'évènement a lieu, elle peut informer une pluralité de personnes (2, 3) de l'évènement (5), qui s'est produit, dans lequel respectivement une unité de commande (6, 7) et au moins un capteur (8, 9) sont prévus pour chaque personne (2, 3), l'unité de commande (6, 7) pouvant être reliée à l'unité d'évaluation (1) pour un échange de données, et au moins un des capteurs (8, 9) mesurant un paramètre vital de la personne respective (2, 3), et si l'unité d'évaluation (1) détermine qu'un évènement s'est produit, au moins une personne (3) de la pluralité de personnes (2, 3) sera sélectionnée sur la base des paramètres vitaux mesurés et seulement la personne sélectionnée (3) sera alertée, **caractérisé en ce qu'**il y a un gyromètre parmi les au moins un capteurs (8, 9), un nombre de changements de direction par intervalle de temps est déterminé par le gyromètre pour déterminer un état à l'éveil ou de sommeil et l'unité d'évaluation (1) alerte la personne (2, 3) parmi les personnes éveillées, qui montre la moindre tension, dans lequel la tension artérielle ou la fréquence du pouls est prise comme base du paramètre vital.

13. Produit de programme d'ordinateur comprenant des commandes, qui, dans le cas d'exécution du programme par un ordinateur, notamment par l'unité d'évaluation, incitent celui-ci à exécuter les étapes du procédé selon la revendication précédente.
